# EUROPÄISCHE PATENTSCHRIFT

(11) **EP 1 280 487 B1**
(45) Veröffentlichungstag und Bekanntmachung des Hinweises auf die Patenterteilung: **21.03.2007**
(21) Anmeldenummer: 01936181.5
(22) Anmeldetag: 04.04.2001
(51) Int. Cl.: A61F 13/06

(54) **BANDAGE FÜR DAS SPRUNGGELENK**
BANDAGE FOR THE ANKLE JOINT
BANDAGE POUR L'ARTICULATION DU PIED

(30) Priorität: 09.05.2000 DE 10022524
(43) Veröffentlichungstag der Anmeldung: 05.02.2003
(73) Patentinhaber: Beiersdorf AG, 20253 Hamburg (DE)
(72) Erfinder: BODENSCHATZ, Stefan, 21614 Buxtehude (DE); ANDREWS, Arthur, Hugh, 25337 Elmshorn (DE)
(86) Internationale Anmeldenummer: PCT/EP2001/003797
(87) Internationale Veröffentlichungsnummer: WO 2001/085078

(56) Entgegenhaltungen:
- DE-A- 19 802 511
- US-A- 4 729 370
- US-A- 5 090 404

## Beschreibung

Die Erfindung betrifft eine Bandage für das Sprunggelenk.

Orthopädischen Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigen Material, zum Beispiel aus Neopren, Gewirke, oder Geweben.
Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.

Das Zusammenfügen kann durch Nähen, Kleben oder andere übliche Verfahren erfolgen. Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Sprunggelenkorthesen oder -bandagen dienen bevorzugt für die frühfunktionelle Behandlung frischer fibularer Bandrupturen und von leichten und mittelschweren Fußwurzeldistorsionen, als auch zur Anwendung bei chronischer Instabilität.

Durch die DE 38 40 714 A1 ist eine Sprunggelenkorthese mit einem U-förmigen Stützbügel bekannt, dessen Schenkel unterhalb des Fußes in einem Steg zusammenlaufen, über die Knöchel hinaufreichen und in ihrem Endbereich durch ein Befestigungsband zusammen gehalten sind. Dabei ist der äußere Schenkel seitlich vor seinem Knöchel und der innere Schenkel in Gegenüberstellung zum äußeren Schenkel vor der Achillessehne hochgeführt. Beide Schenkel sind in Richtung zu dem Steg bis zu einer Lage vor der Ferse geführt und verlaufen in Richtung zu ihren Enden aufwärts derart, daß sie seitlich neben den Schienbeinkanten etwa parallel zu diesen aufwärts streben, wobei im unteren Bereich der Schenkel ein Halteband angebracht ist, das von dem einen Schenkel über den Rist schräg aufwärts zum anderen Schenkel an diesen festlegbar verläuft, oberhalb der Knöchel um die Achillessehne greift und auf den Rist sich überkreuzend an dem anderen Schenkel in einem Halteteil endet. Mit einer derart ausgebildeten Sprunggelenkorthese soll das Umknicken vor allem in Richtung seitvom, also in Richtung auf eine Spitzfuß-Stellung, verhindert werden. Da der U-förmige Stützbügel dieser Sprunggelenkorthese mit seinem äußeren Schenkel seitlich vor dem Fußknöchel und mit seinem inneren Schenkel vor der Achillessehne hochgeführt und von einem unterhalb des Fußes verlaufenden Steg zusammengehalten sind, wird der mediale Rand des Mittelfußes nicht erfaßt, so daß der Einsatz dieser Sprunggelenkorthese begrenzt ist.

Eine Fußfixierungsschiene beschreibt die DE 39 09 922 A1. Diese Fußfixierungsschiene dient insbesondere zur postoperativen Behandlung eines verletzten Sprunggelenkes mit einem den Fuß umfassenden Fußteil, an den sich nach oben bis in den Wadenbereich reichend ein mit Verschlußzügeln versehener Halteteil anschließt. Der Halteteil ist dabei in zwei Seitenteile unterteilt, die an den Fußteil anschließen und schalenförmig ausgebildet sind. Jeweils der den Fußknöchel überdeckende Bereich des Seitenteils ist mit einer fensterartigen Ausnehmung versehen. Der Bereich der Achillessehne am Fußteil und am Halteteil ist dabei ausgespart. Die einstell- und feststellbaren bandförmigen Verschlußzügel bestehen aus einem undehnbaren Material, wobei ein Verschlußzügel an dem Fußteil so angeordnet ist, daß er den Fußrücken übergreifend den ersten Strahl des Mittelfußes gegen supinatorisches Auf steigen fixiert. Mit einer derartigen Fußfixierungsschiene soll zum einen eine einwandfreie Ruhigstellung des zu behandelnden Fußes erreicht werden, und zum anderen sollen die Nachteile eines Gipsverbandes vermieden werden, denn nach Verletzungen und Operationen am äußeren Bandapparat wird oftmals der Fuß zur Ruhigstellung eingegipst, wobei eine postoperative Behandlung von Operationswunden wegen zahlreicher gravierender Nachteile nicht möglich ist. Bei dieser Fußfixierungsschiene wird von einer U-förmigen Gelenkmanschette mit vollflächigem Sohlenteil ausgegangen, das den Mittel- und Vorfuß bis zum kleinen Zehenballen erfaßt, wobei jedoch keine ausreichende Gelenkigkeit im Mittelfußbereich gegeben ist.

Die DE 43 18 588 C2 offenbart ebenfalls eine Sprunggelenkorthese, die aus einer U-förmigen, aus thermoplatischem Material hergestellten Gelenkmanschette besteht, die sich aus einer Außenknöchelschiene und einer Innenknöchelschiene zusammensetzt. Diese Knöchelschienen sind durch einen unter der Ferse verlaufenden Steg verbunden. Die Knöchelschienen weisen ferner anatomisch gerechte Vertiefungen zur Anpassung an die Knöchelkonturen und zum anatomiegerechten Sitz auf. Ein weiteres Element der Orthese ist sein Mittelfußteil, das ebenfalls aus thermoplastischem Material gefertigt ist. Dieser Teil der Orthese läuft quer unter der Fußsohle proximal der Köpfchen der Mittelfußknochen I-V und ist medial und lateral laschenförmig ausgebildet. Die so ausgebildeten Laschen umfassen die Fußränder außen und innen. Sie führen zum einen den Mittelfuß, zum anderen dienen sie zur Befestigung von Kreuz- und Quergurtbändern. Das Mittelfußteil ist dabei fußsohlenseitig durch einen ebenfalls aus thermoplastischem Material hergestellten, jedoch hochflexiblen Steg verbunden. Dieser Steg hat die Funktion eines Gelenkes und arbeitet analog einem Filmscharnier. Die Drehachse dieses in dem hochflexiblen Steg ausgebildeten Gelenkes verläuft von dorso-medial nach antero-lateral und bildet mit der Fußlängsachse einen Winkel von etwa 10°, und zwar entsprechend der Anatomie des unteren Sprunggelenks.

Die DE 34 15 657 C2 betrifft eine Fußgelenkbandage mit einer abgewinkelten, rohrförmigen Knöchelsocke. An der Knöchelsocke ist ein Zugband angebracht.
Es handelt sich bei der Knöchelsocke um einen geschlossenen Körper, der nur für die Fußspitze sowie für das Bein eine Öffnung aufweist. Eine Socke ist vergleichsweise aufwendig und teuer herzustellen. Weiterhin hat sie anwendungstechnische Nachteile, denn sie kann u.a. zu einem Hitzestau am Fuß führen, insbesondere wenn sie aus dem bevorzugt eingesetzten Gummigewebe besteht. Der Hitzestau führt dann zu erhöhter, unangenehmer Schweißbildung.

Die DE 31 22 463 C2 offenbart eine Bandage für das Sprunggelenk, die als zusätzliche Ausstattungsmerkmale zwei beidseitig mit Klettverschlüssen versehene Haftstreifen und zwei Fixier- und Stützstreifen haben muß, um einen guten Sitz am Fuß sowie eine ausreichende Stabilisierung des Gelenks zu gewährleisten.

Mit der DE 43 18 791 C2 ist eine Sprunggelenkbandage bekannt geworden, die als komplex im Aufbau zu bezeichnen ist.
Diese Sprunggelenkbandage besteht aus einem Unterschenkelabschnitt und einem Fußabschnitt, an dem ein Pronationszügel angebracht ist. Der Unterschenkelabschnitt wird aus zwei teilweise zur Überlappung bringbaren Laschen gebildet, die mittels eines Schnellverschlusses zur einer den Unterschenkel umgebenden Röhre ausgebildet werden können.

Die DE 92 11 750 U1 beschreibt eine Sprunggelenkbandage, die sehr wenig praxisrelevant ist, weil diese nur den oberen Teil des Sprunggelenks umgibt und zwingend eine bewegliche Verbindung zu einem zu tragenden Schuh erfordert, die über Bandstrukturen hergestellt wird.

Die aus der WO 92/19187 A1 bekannte Sprunggelenkbandage besteht aus einer Socke, die aus einem entsprechenden Zuschnitt genäht wird, an der ein Zügel befestigt ist. Des weiteren beginnt der Zügel bei angelegter Bandage am Sprunggelenk, nicht, wie bei der erfindungsgemäßen Bandage, an der Fußsohle.

Die US 3,699,959 A zeigt eine Bandage, die aus zwei Streifen besteht, wobei der kürzere fest an dem längeren derartig angenäht ist, daß er den Fuß an der Hacke umgibt. Der längere der beiden Streifen wird dann in einer komplizierten Bewegung kreuzförmig um den Fuß gelegt.

Die US 3,777,751 A zeigt eine Bandage für das Sprunggelenk, die ebenfalls von zwei Zügeln gebildet wird. Der erste der beiden Zügel wird auf sich vemäht, so daß sich ein geschlossener Kreis bildet. Direkt auf die Nahtstelle wird der zweite Zügel ebenfalls durch Aufnähen befestigt. Der zweite Zügel wird schließlich durch einige das Bein umgebende Streifen eines Klebebands am selbigen befestigt.

US-A-5090404 beschreibt eine Bandage für das Sprunggelenk, die aus einem flachen Zuschnitt besteht und eine T-förmigen Unterlage sowie mehrere Zügel umfasst. Vom Vorfuss der angelegten Bandage ausgehende Zügel sind dabei nicht vorgesehen.

Die DE 198 02 511 A1 offenbart eine gattungsgemäße Bandage, die am Sprunggelenk für eine funktionelle Behandlung von leichten und mittelschweren Fußwurzeldistorsionen und chronischen Instabilitäten geeignet ist. Die Bandage besteht aus einem länglichen Steifen und einem an dem länglichen Streifen befestigten Zügel. Die erste Querkante des Streifens ist im wesentlichen vertikal auf der medialen Seite des Sprunggelenks angeordnet. Ausgehend von der ersten Querkante wird der längliche Streifen um die Ferse, über die laterale Seite des Sprunggelenks, über den Fußrücken nach medial plantar und über die Fußsohle nach lateral plantar geführt. Die erste Querkante des länglichen Streifens ist am länglichen Streifen selbst befestigt, und zwar bevorzugt vernäht.
Des weiteren setzt an der zweiten Querkante der Zügel an, der ausgehend von der lateralen Seite der Fußsohle über den Fußrücken zur medialen Seite des Sprunggelenks, um die Ferse zur lateralen Seite des Sprunggelenks und über die laterale Seite des Sprunggelenks hinaus geführt und befestigt wird.

Der Erfindung liegt die Aufgabe zugrunde, eine Bandage zu schaffen, die insbesondere am Sprunggelenk einzusetzen und die als solche für eine funktionelle Behandlung von leichten und mittelschweren Fußwurzeldistorsionen und chronischen Instabilitäten geeignet ist, mit der eine gezielte laterale und mediale Stabilisierung vom oberen und unteren Sprunggelenk erreicht wird, wodurch das Risiko sowohl für Inversionstraumata als auch Eversionstraumata verringert wird und die für den Patienten besonders einfach und sicher in der Anwendung ist.

Diese Aufgabe wird durch die gemäß Hauptanspruch gekennzeichnete Bandage gelöst. Gegenstand der Unteransprüche sind dabei vorteilhafte Weiterbildungen der Bandage.

Demgemäß betrifft die Erfindung eine Bandage für das Sprunggelenk, bestehend aus einem länglichen Band, wobei das längliche Band um die Ferse, über die laterale Seite des Sprunggelenks, über den Fußrücken nach medial plantar und über die Fußsohle nach lateral plantar geführt wird, wobei die erste und die zweite Querkante des Bandes am Band selbst befestigt sind. Am länglichen Band setzen zwei Zügel an, ein erster ausgehend von der lateralen Seite am Vorfuß und ein zweiter von der lateralen Seite am Sprunggelenk, wobei der zweite Zügel zirkulär um das Sprunggelenk geschlossen wird und der erste Zügel am Vorfuß über den Fußrücken zur medialen Seite des Sprunggelenks geführt und zirkulär um das Sprunggelenk geschlossen wird.
Das längliche, elastische Band ergibt somit die Form einer geschlossenen Acht.

Das Material für das längliche Band weist bevorzugt eine Längselastizität von 60% bis 250%, insbesondere 80% bis 140%, und eine Querelastizität von bis zu 100%, insbesondere 5% bis 30%, auf.

Die Zügel können aus elastischen klettfähigen textilen Bändern hergestellt werden, jedoch auch z.B. aus Neoprenschaum. Das Material der Zügel hat in einer weiteren vorteilhaften Ausführungsform der Bandage eine Längselastizität von bis 250%, insbesondere 30% bis 100%, und eine Querelastizität von bis zu 100%, insbesondere 5% bis 30%.

Die Umfassung des Fußes mit dem elastischen Band führt zu einer Kompressionswirkung. Die Form des elastischen Bandes, die Acht, folgt den anatomischen Gegebenheiten. Mit dem zirkulären Zügel am oberen Abschluß der Bandage läßt sich die Kompressionswirkung individuell einstellen und somit eine individuelle laterale und mediale Stabilisierung vom oberen und unteren Sprunggelenk erreichen.

Der individuell einstellbare laterale Zügel, vom Vorfuß kommend und auf dem Fußrücken zum Sprunggelenk verlaufend, verringert durch die laterale und mediale Stabilisierung das Risiko sowohl für Inversionstraumata als auch für Eversionstraumata

Da beide Zügel individuell verstellbar sind, ist es möglich je nach Indikation die Kompression und Unterstützungkraft entsprechend einzustellen. Trotz guter Stabilisierung ermöglicht die Bandage das physiologische Gangbild.

Die Bandage passt universell für den rechten und linken Fuß. Die Bandage ist jeweils so anzulegen, daß die Zügel lateral ansetzen.

Die Bandage wird ähnlich einer Socke angezogen. Anschließend wird zunächst der zirkuläre Gurt geschlossen, anschließend der laterale Gurt. Somit ist die Bandage für den Patienten einfach anzulegen. Aufgrund der Aussparung der Fersenpartie ist die Bandage einfach und sicher am Fuß in der richtigen Position zu plazieren

Die Zügel der Bandage sind mit Klettverschlüssen versehen, können aber auch mittels Druckknöpfen etc. geschlossen werden.

Als Material für die Bandage wird ein hautfreundliches Material eingesetzt. So können textile Bänder, jedoch auch zum Beispiel Neoprenschaum verwendet werden. Besonders vorteilhaft können Abstandsgewebe verwendet werden, die besonders atmungsaktiv sind.

Die Erfindung wird anhand einer schematischen Zeichnung eines Ausführungsbeispiels näher erläutert, und zwar angelegt am rechten Fuß. Die Ausführungsvariante der Bandage für den linken Fuß ist zu der ersteren spiegelsymmetrisch ausgeführt. In der bevorzugten Ausführungsform kann durch Umstülpen der Bandage diese von der rechten in die linke Ausführungsvariante überführt werden.

Das elastische Band 1 ist mit den beiden Stirnseiten 2 an sich selbst befestigt, so daß eine Socke 3 in Form einer Acht gebildet ist. Die Bandage wird auf den Fuß 10 angezogen, so daß die Zügel lateral zu liegen kommen und die Ferse 11 in der Öffnung 4 positioniert ist. Anschließend wird der obere Zügel 5 zirkulär um das Sprunggelenk 6 mit dem angenähten Klettband 9 auf sich selbst geschlossen. Der laterale untere Zügel 7 wird über den Fußrücken 8 geführt und ebenfalls zirkulär um das Sprunggelenks 6 mit dem angebrachten Klettband 9 auf sich selbst geschlossen.

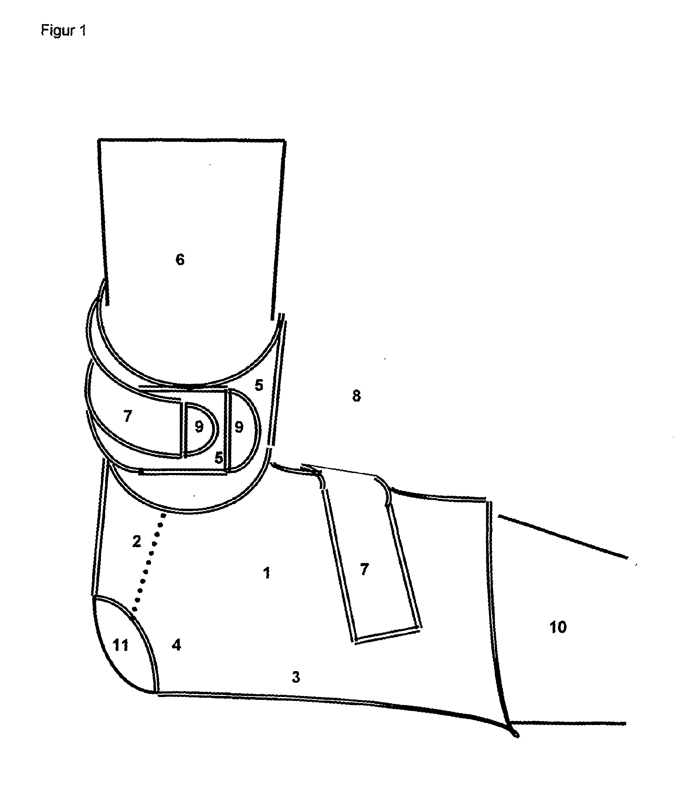

## Patentansprüche

1. Bandage für das Sprunggelenk, bestehend aus einem länglichen Band (1), wobei das längliche Band (1) um die Ferse (11), über die laterale Seite des Sprunggelenks, über den Fußrücken (8) nach medial plantar und über die Fußsohle nach lateral plantar geführt wird, wobei die erste und die zweite Querkante des Bandes (1) am Band (1) selbst befestigt sind (2) und wobei am länglichen Band zwei Zügel (5, 7) ansetzen,
**dadurch gekennzeichnet, dass**
ein erster Zügel (5 oder 7) von der lateralen Seite am Vorfuß und ein zweiter (7 oder 5) von der lateralen Seite am Sprunggelenk ausgehen, wobei der zweite Zügel zirkulär um das Sprunggelenk geschlossen wird und der erste Zügel am Vorfuß über den Fußrücken zur medialen Seite des Sprunggelenks geführt und zirkulär um das Sprunggelenk geschlossen wird.

2. Bandage nach Anspruch 1, **dadurch gekennzeichnet, dass** das Material für das längliche Band (1) eine Längselastizität von 60% bis 250%, insbesondere 80% bis 140%, und eine Querelastizität von bis zu 100%, insbesondere 5% bis 30%, hat.

3. Bandage nach den Ansprüchen 1 und 2, **dadurch gekennzeichnet, dass** das Material der Zügel (5, 7) eine Längselastizität von bis 250%, insbesondere 30% bis 100%, und eine Querelastizität von bis zu 100%, insbesondere 5% bis 30%, hat.

4. Bandage nach den Ansprüchen 1 bis 3, **dadurch gekennzeichnet, dass** die Zügel (5, 7) mit einem am Ende angebrachten Klettband (9) auf sich selbst geschlossen werden können.

5. Bandage nach den Ansprüchen 1 bis 4, **dadurch gekennzeichnet, dass** die Bandage durch Drehen um 180 Grad von der rechten in die linke Ausführungsform überführt werden kann.

## Claims

1. Bandage for the ankle joint, comprising an elongate tape (1), said elongate tape (1) being guided round the heel (11), across the lateral side of the ankle joint, across the instep (8) in the medial-plantar direction and across the sole of the foot in the lateral-plantar direction, the first and second transverse edges of the tape (1) being secured (2) on the tape (1) itself, and two straps (5, 7) being joined to the elongate tape, **characterized in that** a first strap (5 or 7) starts from the lateral side of the forefoot, and a second strap (7 or 5) starts from the lateral side of the ankle joint, the second strap being closed in a circle round the ankle joint, and the first strap on the forefoot being guided across the instep to the medial side of the ankle joint and being closed in a circle round the ankle joint.

2. Bandage according to Claim 1, **characterized in that** the material for the elongate tape (1) has a longitudinal elasticity of 60% to 250%, in particular 80% to 140%, and a transverse elasticity of up to 100%, in particular 5% to 30%.

3. Bandage according to Claims 1 and 2, **characterized in that** the material of the straps (5, 7) has a longitudinal elasticity of up to 250%, in particular 30% to 100%, and a transverse elasticity of up to 100%, in particular 5% to 30%.

4. Bandage according to Claims 1 to 3, **characterized in that** the straps (5, 7) can be closed on themselves with a velcro tape (9) attached at the end.

5. Bandage according to Claims 1 to 4, **characterized in that**, by turning the bandage through 180 degrees, it can be converted from the right-foot version to the left-foot version.

## Revendications

1. Bandage pour l'articulation de la cheville, constitué d'une bande allongée (1), la bande allongée (1) étant passée autour du talon (11), sur le côté latéral de l'articulation de la cheville, sur le dos (8) du pied en direction médiale plantaire et sur la plante du pied en direction latérale plantaire, le premier et le deuxième bord transversal de la bande (1) étant fixés (2) de même sur la bande (1), deux brides (5, 7) se posant sur la bande allongée,
**caractérisé en ce que**
une première bride (5 ou 7) part du côté latéral de l'avant du pied et une deuxième bride (7 ou 5) du côté latéral de l'articulation de la cheville, la deuxième bride étant fermée en cercle autour de l'articulation de la cheville et la première bride étant passée sur l'avant du pied au-dessus du dos du pied en direction du côté médial de l'articulation de la cheville et étant fermée en cercle autour de l'articulation de la cheville.

2. Bandage selon la revendication 1, **caractérisé en ce que** le matériau de la bande allongée (1) présente une élasticité longitudinale comprise entre 60 % et 250 % et en particulier entre 80 % et 140 % et une élasticité transversale qui peut atteindre 100 et en particulier comprise entre 5 % et 30 %.

3. Bandage selon les revendications 1 et 2, **caractérisé en ce que** le matériau des brides (5, 7) présente une élasticité longitudinale qui peut atteindre 250 % et qui est en particulier comprise entre 30 % et 100 % et une élasticité latérale qui peut atteindre 100 % et qui est en particulier comprise entre 5 % et 30 %.

4. Bandage selon les revendications 1 à 3, **caractérisé en ce que** les brides (5, 7) peuvent être fermées sur elles-mêmes par une bande d'accrochage élastique (9) installée à leur extrémité.

5. Bandage selon les revendications 1 à 4, **caractérisé en ce que** le bandage peut être passé d'un mode de réalisation droit à un mode de réalisation gauche par rotation de 180 degrés.
